# EUROPÄISCHE PATENTSCHRIFT

(11) **EP 2 066 711 B1**
(45) Veröffentlichungstag und Bekanntmachung des Hinweises auf die Patenterteilung: **29.12.2010**
(21) Anmeldenummer: 07802127.6
(22) Anmeldetag: 05.09.2007
(51) Int. Cl.: C08G 18/08, C08G 18/12, C08J 9/30, C08J 9/00, A61L 15/26

(54) **ALKYLPOLYGLYCOSIDE ALS STABILISATOREN FÜR PUR-SCHÄUME**
ALKYLPOLYGLYCOSIDES AS STABILIZERS FOR PU FOAMS
ALKYLPOLYGLYCOSIDES UTILISÉS COMME STABILISANTS DE MOUSSES DE POLYURÉTHANE

(30) Priorität: 16.09.2006 DE 102006043589
(43) Veröffentlichungstag der Anmeldung: 10.06.2009
(73) Patentinhaber: Bayer MaterialScience AG, 51368 Leverkusen (DE)
(72) Erfinder: MAGER, Michael, 51375 Leverkusen (DE); SCHÖNBERGER, Jan, 42655 Solingen (DE)
(86) Internationale Anmeldenummer: PCT/EP2007/007717
(87) Internationale Veröffentlichungsnummer: WO 2008/031520

(56) Entgegenhaltungen:
- EP-A- 0 246 723
- EP-A- 1 647 562
- WO-A-2007/115697

## Beschreibung

Die Erfindung betrifft Zusammensetzungen zur Herstellung von hydrophilierten Polyurethan-Schäumen, insbesondere für die Wundbehandlung, bei welchem eine Zusammensetzung, enthaltend eine Polyurethan-Dispersion und spezielle Additive, aufgeschäumt und getrocknet wird.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in Regel Polyurethan-Schäume eingesetzt, welche durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind.

Eine Alternative zum vorstehend beschriebenen Verfahren, in welchem Diisocyanate bzw. NCO-funktionelle Polyurethan-Prepolymere eingesetzt werden, ist ein Verfahren basierend auf Polyurethan-Dispersionen (die im wesentlichen frei von Isocyanatgruppen sind), in welche man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen in EP-A 0 235 949 und EP-A 0 246 723 beschrieben, wobei dem Schaum entweder ein selbsthaftendes Polymer zugesetzt oder der Schaum auf einen Film eines selbsthaftenden Polymers aufgebracht wird. In US 4,655,210 ist die Verwendung der vorgenannten mechanischen Schäume für Wundauflagen beschrieben, die einen speziellen Aufbau aus Träger, Schaum und Hautkontakt-Schicht aufweisen. Wie in EP-A 0 235 949, EP-A 0 246 723 und US 4,655,210 beschrieben, wurden zur Herstellung der Schäume aus den Polyurethan-Dispersionen stets Additiv-Mischungen eingesetzt, welche im wesentlichen Ammoniumstearat enthalten. Dies ist jedoch von großem Nachteil, da Ammoniumstearat zu einer deutlichen Hydrophobierung der Schäume führt und dadurch die Aufnahmegeschwindigkeit von Flüssigkeit erheblich vermindert wird. Dies ist insbesondere bei Wundauflage-Schäumen nicht akzeptabel. Darüber hinaus ist Ammoniumstearat thermisch zersetzlich, und der gebildete Ammoniak muß mit entsprechendem technischen Aufwand entsorgt werden. Andererseits hat sich gezeigt, dass Ammoniumstearat nicht einfach durch andere Stearate oder gänzlich andere (Schaum-) Additive ersetzt werden kann, da keine vergleichbar gute Schaumstruktur, gekennzeichnet vor allem durch sehr feine Poren, erhalten wird.

Aufgabe der vorliegenden Erfindung war daher die Bereitstellung von geeigneten (Schaum-) Additiven, welche in Kombination mit wässrigen Polyurethandispersionen aufgeschäumt werden können und nach Trocknung feinporige und auch in großen Schichtdicken homogene Schäumen liefern, die verglichen mit ammoniumstearatstabilisierten Schäumen eine verbesserte Hydrophilie und verbunden damit eine schnelle und gute Wasseraufnahme sowie Wasserdampfdurchlässigkeit aufweisen und überdies weitestgehend frei von (thermisch) abspaltbaren Komponenten wie Aminen sind.

Es wurde nun gefunden, dass die zugrunde liegende Aufgabe durch Alkylpolyglycoside als (Schaum-)Additiv gelöst werden kann.

Gegenstand der Erfindung ist daher die Verwendung von Alkylpolyglycosiden als Stabilisatoren für Polyurethanschäume. Bevorzugt wird durch die erfindungsgemäße Verwendung neben der Stabilisierung auch eine zusätzliche Hydrophilierung der Schäume erzielt. Bevorzugt handelt es sich bei den vorgenannten Polyurethanschäumen um solche, wie sie durch physikalische Trocknung aus wässrigen Polyurethandispersionen erhalten werden.

Gegenstand der Erfindung ist ferner ein Verfahren zur Herstellung von Polyurethan-Schäumen, bei dem eine Zusammensetzung, welche ebenfalls Gegenstand der Erfindung ist, enthaltend wässrige, anionisch hydrophilierte Polyurethan-Dispersionen (I) und Additive (II) aufgeschäumt und getrocknet wird, wobei die Schaumadditive (II) wenigstens ein Alkylpolyglycosid umfassen.

Die in den erfindungswesentlichen Zusammensetzungen enthaltenen wässrigen, anionisch hydrophilierten Polyurethan-Dispersionen (I) sind erhältlich, indem
(1) isocyanatfunktionelle Prepolymere mindestens aus
   - A1): organischen Polyisocyanaten
   - A2): polymeren Polyolen mit zahlenmittleren Molekulargewichten von 400 bis 8000 g/mol und OH-Funktionalitäten von 1,5 bis 6 und
   - A3): gegebenenfalls hydroxyfunktionellen Verbindungen mit Molekulargewichten von 62 bis 399 g/mol und
   - A4): gegebenenfalls isocyanatreaktiven, anionischen oder potentiell anionischen und/oder gegebenenfalls nichtionischen Hydrophilierungsmitteln,
   hergestellt werden,
(2) deren freie NCO-Gruppen dann ganz oder teilweise
   - B1): gegebenenfalls mit aminofunktionellen Verbindungen mit Molekulargewichten von 32 bis 400 g/mol und/oder
   - B2): isocyanatreaktiven, bevorzugt aminofunktionellen, anionischen oder potentiell a- nionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

Bedeutsam dabei ist, dass die Verbindungen der Komponenten A1) bis A4) keine primären oder sekundären Aminogruppen aufweisen.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder -SO₃⁻ oder -PO₃²⁻ als anionische bzw. deren ganz oder teilweise protonierte Säureformen als potentiell anionische Gruppen aufweisen.

Bevorzugte wässrige, anionische Polyurethan-Dispersionen (I) haben einen niedrigen Grad an hydrophilen anionischen Gruppen, bevorzugt von 0,1 bis 15 Milliequivalenten pro 100 g Festharz.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 550 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 bis 125 %, besonders bevorzugt zwischen 60 bis 120 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisoeyanat, 1,4-Phenylendiisocyanat, 2,4-und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mₙ bevorzugt von 400 bis 6000 g/mol und besonders bevorzugt von 600 bis 3000 g/mol eingesetzt. Diese weisen bevorzugt eine OH-Funktionalität von 1,8 bis 3, besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol bevorzugt 1,6-Hexandiol und/oder Hexandiolderivate bezogen auf die zugrunde liegenden Diole. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung 20 bis 80 Gew.-% und der Anteil an Polytetramethylenglykolpolyolen 80 bis 20 Gew.-% beträgt. Bevorzugt ist ein Anteil von 30 bis 75 Gew.-% an Polytetramethylenglykolpolyolen und ein Anteil von 25 bis 70 Gew.-% an Polycarbonatpolyolen. Besonders bevorzugt ist ein Anteil von 35 bis 70 Gew.-% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.-% bevorzugt 60 Gew.-% und besonders bevorzugt mindestens 70 Gew.-% beträgt.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-e-hydroxycapronsäureester, ω-Hydroxyhexyl-γ-hydroxybuttersäureester, Adipinsäure-(β-hydroxyethyl)ester oder Terephthalsäurebis(β-hydroxyethyl)ester.

Ferner können in A3) auch monofunktionelle, hydroxylgruppen-haltige Verbindungen eingesetzt werden. Beispiele solcher monofunktionellen Verbindungen sind Ethanol, n-Butanol, Ethylenglykolmonobutylether, Diethylenglykolmonomethylether, Ethylen-glykolmonobutylether, Diethylenglykolmonobutylether, Propylenglykolmonomethylether, Dipropylenglykol-monomethylether, Tripropylenglykolmonomethylether, Dipropylenglykol-monopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO⁻M⁺, -SO₃⁻ M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, ,̅ Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel der Komponente A4) sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S.31-38).

Diese sind entweder reine Polyethylenoxidether oder gemischte Polyalkylenoxidether, wobei sie dann aber mindestens 30 mol%, bevorzugt mindestens 40 mol% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol% Ethylenoxid- und 0 bis 60 mol% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin oder sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)anünopropylarnin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin.

Unter anionisch bzw. potentiell anionisch hydrophilierenden Verbindungen der Komponente B2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻M⁺, - SO₃⁻M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Aminoethylamino)ethansulfonsäure, Ethylendiaminpropyl- oder -butylsulfonsäure, 1,2- oder 1,3-Propylendiamin-β-ethylsulfonsäure, Glycin, Alanin, Taurin, Lysin, 3,5-Diarninobenzoesäure und das Additionsprodukt von IPDA und Acrylsäure (EP-A 0 916 647, Beispiel 1).,̅ Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser oder gelöster (homogener) Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen 1,05 bis 3,5, bevorzugt 1,2 bis 3,0, besonders bevorzugt 1,3 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar. Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene organischen Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (I) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (I) beträgt typischerweise 40 bis 70, bevorzugt 50 bis 65, besonders bevorzugt 55 bis 65 Gew.-%.

Die in den Schaumadditiven (II) enthaltenen Alkylpolyglycoside sind nach dem Fachmann an sich bekannten Methoden durch Umsetzung von längerkettigen Monoalkoholen mit Mono-, Di- oder Polysacchariden erhältlich (Kirk-Othmer Encyclopedia of Chemical Technology, John Wiley & Sons, Vol. 24, S. 29). Die längerkettigen Monoalkohole, die gegebenenfalls auch verzweigt sein können, weisen bevorzugt 4 bis 22 C-Atome, bevorzugt 8 bis 18 C-Atome und besonders bevorzugt 10 bis 12 C-Atome im Alkylrest auf. Im Einzelnen genannt seien als längerkettige Monoalkohole 1-Butanol, 1-Propanol, 1-Hexanol, 1-Oktanol, 2-Ethylhexanol, 1-Decanol, 1-Undecanol, 1-Dodecanol (Laurylalkohol), 1-Tetradecanol (Myristylalkohol) und 1-Octadecanol (Stearylalkohol). Selbstverständlich können auch Gemische der genannten längerkettigen Monoalkohole eingesetzt werden.

Bevorzugt weisen diese Alkylpolyglycoside von der Glucose abgeleitete Strukturen auf.

Besonders bevorzugt werden Alkylpolyglycoside der Formel (I) eingesetzt.

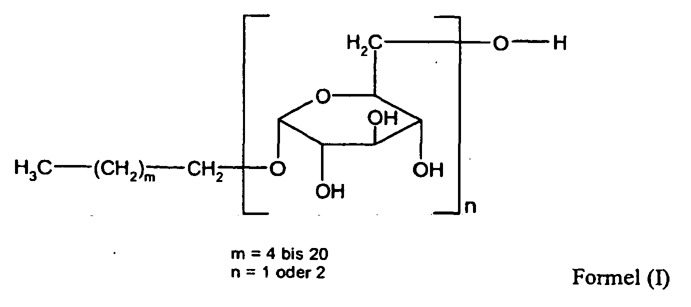

Bevorzugt ist m eine Zahl von 6 bis 20, besonders bevorzugt 10 bis 16.

Die Alkylpolyglycoside weisen bevorzugt einen HLB-Wert von weniger als 20, besonders bevorzugt von weniger als 16 und ganz besonders bevorzugt von weniger als 14 auf, wobei sich der HLB-Wert nach der Formel HLB = 20 · Mh/M errechnet, wobei Mh die Molmasse des hydrophilen Anteils eines Moleküls und M die Molmasse des gesamten Moleküls ist (Griffin, W.C.: Classification of surface active agents by HLB, J. Soc. Cosmet. Chem. 1, 1949).

Zur Verbesserung der Schaumbildung, Schaumstabilität oder der Eigenschaften des resultierenden Polyurethan-Schaums, können neben den Alkylpolyglycosiden noch weitere Additive in der Komponente (II) enthalten sein.

Solche weiteren Additive können grundsätzlich alle an sich bekannten anionischen, nichtionischen und kationischen Tenside sein. Bevorzugt ist jedoch, dass neben den Alkylpolyglycosiden auch Ester der Sulfobernsteinsäure, wobei der lipophile Alkylrest der Estergruppe bevorzugt 8 bis 24 Kohlenstoffatome enthält, und/oder Alkali- oder Erdalkalimetallalkanoate eingesetzt werden, in welchen der lipophile Alkylrest bevorzugt 12 bis 24 Kohlenstoffatome enthält. Besonders bevorzugt werden als weitere Additive sowohl Ester der Sulfobemsteinsäure als auch Alkali- oder Erdalkalimetallalkanoate der vorstehend genannten Art eingesetzt.

Überdies können als weitere Additive sogar Ammoniumalkanate wie Ammoniumstearat zugesetzt werden, da die hydrophilierende Wirkung der Alkylpolyglycoside erhalten bleibt. Die weiteren Additive werden bevorzugt in geringer Menge als die Alkylpolyglycoside eingesetzt.

Neben den Polyurethan-Dispersionen (I) und den Schaumadditiven (II) können auch weitere Hilfs- und Zusatzstoffe (III) mitverwendet werden.

Beispiele für solche Hilfs- und Zusatzstoffe (III) sind Vernetzer, Verdicker bzw. Thixotropiermittel, andere wässrige Bindemittel, Antioxidantien, Lichtschutzmittel, Emulgatoren, Weichmacher, Pigmente, Füllstoffe und/oder Verlaufshilfsmittel.

Als Vernetzer können beispielsweise zugesetzt werden unblockierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten sein.

Als Verdicker können handelsübliche Verdicker eingesetzt werden, wie Dextrin-, Stärke- oder Cellulosederivate wie Celluloseether- oder Hydroxyethylcellulose, organische vollsynthetische Verdicker, auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Andere wässrige Bindemittel können z. B. aus Polyester-, Polyacrylat-, Polyepoxid- oder anderen Polyurethanpolymeren aufgebaut sein. Auch die Kombination mit strahlenhärtbaren Bindemitteln, wie sie z. B. in der EP-A-0 753 531 beschrieben sind, ist möglich. Ferner können auch andere anionische oder nicht-ionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Die erfindungswesentlichen Zusammensetzungen enthalten, bezogen auf Trockensubstanz, typischerweise 80 bis 99,9 Gewichtsteile Polyurethan (aus der Polyurethan-Dispersion(I)) und 0,1 bis 20 Gewichtsteile des Schaumadditivs (II). Bevorzugt enthalten die Zusammensetzungen, bezogen auf Trockensubstanz, 85 bis 99,5 Gewichtsteile Polyurethan und 0,5 bis 15 Gewichtsteile des Schaumadditivs (II), besonders bevorzugt 90 bis 99 Gewichtsteile des Polyurethan und 1 bis 10 Gewichtsteile des Schaumadditivs (II), ganz besonders bevorzugt 97,5 bis 99 Gewichtsteile Polyurethan und 1 bis 2,5 Gewichtsteile des Schaumadditivs (II).

Die weiteren als Hilfs- und Zusatzstoffe (III) zugegebenen Additive werden typischerweise in Mengen von 0 bis 10 Gewichtsteilen, bevorzugt 0,1 bis 5 Gewichtsteilen und besonders bevorzugt 0,1 bis 1,5 Gewichtsteilen zu der erfindungsgemäßen Zusammensetzung eingesetzt.

Die Zugabe der Schaumadditive (II) und der gegebenenfalls ebenso einzusetzenden weiteren Additive zur Polyurethan-Dispersion kann in beliebiger Reihenfolge erfolgen. Die vorgenannten Additive können dabei gegebenenfalls in einem Lösungsmittel wie Wasser gelöst oder dispergiert eingesetzt werden.

Grundsätzlich ist es auch möglich im Rahmen der Hilfs- und Zusatzstoffe durch Zugabe von Koagulantien eine Koagulation des Schaums herbeizuführen. Hierzu sind prinzipiell alle mehrfach kationisch funktionellen Verbindungen geeignet.

Das Aufschäumen im erfindungsgemäßen Verfahren kann durch Schütteln der Zusammensetzung, mechanisches Rühren oder durch Entspannung eines Treibgases erfolgen.

Das mechanische Aufschäumen kann mit beliebigen mechanischen Rühr-, Misch- und Dispergiertechniken unter Eintrag der zur Aufschäumung notwendigen Energie erfolgen. In der Regel wird hierbei Luft eingetragen, aber auch Stickstoff und andere Gase können hierfür benutzt werden.

Der so erhaltene Schaum wird beim Aufschäumen oder danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet. Für eine schnellere Trocknung und Fixierung der Schäume werden jedoch bevorzugt Temperaturen oberhalb von 30°C benutzt. Bei der Trocknung sollten jedoch Temperaturen von 200°C nicht überschritten werden, da es anderenfalls u.a. zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlem. Auch die Trocknung durch Führen des beschichteten Substrates über geheizte Oberflächen, z.B. Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren.

Als Substrate geeignet sind insbesondere Papiere oder Folien, die ein einfaches Ablösen der Schäume vor ihrem Einsatz z.B. als Wundauflage zur Abdeckung einer verletzten Stelle ermöglichen.

Die Polyurethan-Schäume haben vor ihrer Trocknung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Polyurethan-Schäume besitzen nach ihrer Trocknung eine mikroporöse, zumindest teilweise offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³ und liegt ganz besonders bevorzugt bei 0,1 bis 0,3 g/cm³.

Durch die Verwendung der speziellen Additive (II) wird eine sehr rasche Aufnahme von Flüssigkeit, insbesondere von physiologischer Salzlösung, erreicht. In der Regel wird 1 ml der Prüflösung A, hergestellt nach DIN EN 13726-1, Teil 3.2, in weniger als 25 Sekunden, bevorzugt in weniger als 10 Sekunden und ganz besonders bevorzugt in weniger als 3 Sekunden vollständig aufgenommen.

Das Absorptionsvermögen gegenüber physiologischer Salzlösung beträgt bei den Polyurethan-Schäumen typischerweise 100 und 1500 %, bevorzugt 300 bis 800 % (Masse der aufgenommen Flüssigkeit bezogen auf die Masse des trockenen Schaums; Bestimmung nach DIN EN 13726-1, Teil 3.2). Die Durchlässigkeit gegenüber Wasserdampf beträgt typischerweise 2000 bis 8000 g/24 h * m², bevorzugt 3000 bis 8000 g/24 h * m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,2 N/mm² und die maximale Dehnung ist größer als 250 %. Bevorzugt ist die maximale Spannung größer als 0,4 N/mm² und die Dehnung größer als 350 % (Bestimmung nach DIN 53504).

Die Polyurethan-Schäume haben nach dem Trocknen typischerweise eine Dicke von 0,1 mm bis 50 mm, bevorzugt 0,5 mm bis 20 mm, besonders bevorzugt 1 bis 10 mm, ganz besonders bevorzugt 1 bis 5 mm.

Die Polyurethan-Schäume können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Den Polyurethan-Schäumen können überdies Wirkstoffe zugegeben werden, die beispielsweise einen Einfluss auf die Wundheilung haben.

Aufgrund ihrer vorteilhaften Eigenschaften werden die erfindungsgemäßen Polyurethan-Schäume bevorzugt als Wundauflagen oder für kosmetische Zwecke eingesetzt. Wundauflagen aus Polyurethan-Schäumen im Sinne der Erfindung sind poröse Materialien, bevorzugt mit zumindest teilweise vorhandener Offenzelligkeit, welche im wesentlichen aus Polyurethanen bestehen und Wunden im Sinne einer sterilen Abdeckung vor Keimen bzw. Umgebungseinflüssen schützen, eine schnelle und hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Ein weiterer Gegenstand der vorliegenden Erfindung sind daher die nach dem erfindungsgemäßen Verfahren erhältlichen Polyurethan-Schäume, sowie deren Verwendung als Wundauflage sowie im kosmetischen Bereich. Bevorzugt ist die Verwendung als Wundauflage.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

### Verwendete Substanzen und Abkürzungen:

- Diaminosulfonat:: NH₂-CH₂CH₂-NH-CH₂CH₂-SO₃Na (45 %ig in Wasser)
- Desmophen^{®} C2200:: Polycarbonatpolyol, OH-Zahl 56 mg KOH/g, zahlerimittleres Molekular- gewicht 2000 g/mol (BayerMaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlen- mittleres zahlenmittleres Molekulargewicht 1000 g/mol (BASF AG, Ludwigshafen, DE)
- Polyether LB 25:: monofunktioneller Polyether auf Ethylenoxid-/Propylenoxidbasis, zah- lenmittleres Molekulargewicht 2250 g/mol, OH-Zahl 25 mg KOH/g (BayerMaterialScience AG, Leverkusen, DE)
- Impranil^{®} DLU: aliphatische Polycarbonatpolyetherpolyurethan-Dispersion, Feststoffge- halt 60 %, ph-Wert 8,0 (BayerMaterialScience AG, Leverkusen, DE)

Die Bestimmung der mittleren Teilchengrößen (angegeben ist das Zahlenmittel) der Polyurethan-Dispersion 1 erfolgte mittels Laserkorrelations-Spektroskopie (Gerät: Malvern Zetasizer 1000, Malver Inst. Limited).

Die Bestimmung der "freien Saugleistung" erfolgte durch Absorption physiologischer Salzlösung nach DIN EN 13726-1, Teil 3.2. Die MVTR ("moisture vapour transmission rate", Wasserdampfdurchlässigkeit) wurde bestimmt nach DIN EN 13726-2, Teil 3.2.

Die für die Schaumadditive angegebenen Gehalte beziehen sich auf wässrige Lösungen.

### Beispiel 1: Herstellung der Polyurethan-Dispersion 1

1077,2 g PolyTHF^{®} 2000, 409,7 g PolyTHF^{®} 1000, 830,9 g Desmophen^{®} C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:
- Feststoffgehalt:: 61,6 %
- Partikelgröße (LKS):: 528 nm
- pH (23°C):: 7,5

### Vergleichsbeispiele V1-V10: Herstellung von Schäumen aus der Polyurethan-Dispersion 1 und Impranil^{®} DLU

Wie in Tabelle 1 angegeben, wurde die Polyurethan-Dispersion 1, hergestellt nach Beispiel 1, bzw. Impranil^{®} DLU mit verschiedenen (Schaum-) Additiven vermischt und unter Verwendung eines handelsüblichen Handrührgerätes (Rührer aus gebogenem Draht) auf 0,5 bzw. 1 Liter Schaumvolumen aufgeschlagen. Danach wurden die Schäume mittels eines Filmziehgerätes (Rakel) mit Spalthöhe 6 mm auf nicht-haftendes Papier aufgezogen und unter den angegebenen Bedingungen getrocknet.

Nur mit den Additiv-Kombinationen enthaltend Ammoniumstearat, Vergleichsversuche V1, V2, V3 und V 10, konnten Schäume erhalten werden, welche für eine weitere Prüfung geeignet waren. Wie der Tabelle 2 zu entnehmen ist, zeigten diese Schäume jedoch eine starke Hydrophobierung und somit sehr geringe Aufnahmegeschwindigkeit von physiologischer Salzlösung (alle > 60 s bzw. > 20 s). Die Wasserdampfdurchlässigkeit (MVTR) ist vergleichsweise niedrig. Mit allen anderen Additiven (Vergleichsversuche V4-V8) konnten überhaupt keine Schäume hergestellt werden (zu geringes Schaumbildungsvermögen der Additive).

**Tabelle 1**

| | | (Schaum-) Additive | | | | |
|---|---|---|---|---|---|---|
| Schaum Nr. | Polyurethan-Dispersion 1 | Typ³⁾ | Menge [g] | Typ³⁾ | Menge [g] | Aushärtung |
| V1 | 235,0¹⁾ | A | 8,5 | B | 11,3 | 60 min 60 °C, 10 min 120 °C |
| V2 | wie bei V1 | | | | | 30 min 60 °C, 10 min 120 °C |
| V3 | 235,0¹⁾ | A | 8,5 | C | 0,9 | 60 min 60 °C, 10 min 120 °C |
| V4 | 117,5²⁾ | C | 0,5 | D | 2,5 | |
| V5 | 117,5²⁾ | C | 0,5 | E | 2,5 | |
| V6 | 117,5²⁾ | C | 0,5 | F | 2,5 | |
| V7 | 117,5²⁾ | C | 0,5 | G | 2,5 | |
| V8 | 117,5²⁾ | C | 0,5 | H | 2,5 | |
| V9 | 117,5²⁾ | C | 0,5 | 1 | 2,5 | |
| | | | | | | |

| | | (Schaum-) Additive | | | | |
|---|---|---|---|---|---|---|
| Schaum Nr. | Impranil^{®} DLU | Typ³⁾ | Menge [g] | Typ³⁾ | Menge [g] | Aushärtung |
| V10 | 117,5²⁾ | A | 4,2 | B | 5,6 | 10 min 120 °C |

| | | | | | | |
|---|---|---|---|---|---|---|
| ¹⁾ Schaumvolumen 1000 ml; ²⁾ Schaumvolumen 500 ml; ³⁾ A: Ammoniumstearat (ca. 30 %, Stokal^{®} STA, Bozzetto GmbH, Krefeld, DE); B: Sulfosuccinarnat (ca. 34 %, Stokal^{®} SR, Bozzetto GmbH, Krefeld, DE); C: Sulfobernsteinsäu-rebis(2-ethylhexylester), Na-Salz; D: Alkylarylpolyglykolethersulfat, Na-Salz (Disponil^{®} AES 25, Cognis Deutschland GmbH & Co.KG, Düsseldorf, DE); E: modifizierter Fettalkoholpolyglykolether (ca. 75 %, Disponil^{®} AFX 2075, Cognis Deutschland GmbH & Co.KG, Düsseldorf, DE); F: Fettalkoholpolyglykolethersulfat, Na-Salz (Disponil^{®} FES 61, Cognis Deutschland GmbH & Co.KG, Düsseldorf, DE); G: Fettalkoholpolyglykolethersulfat, Na-Salz (Disponil^{®} FES 993, Cognis Deutschland GmbH & Co.KG, Düsseldorf, DE); H: C₁₃-Fettalkoholethoxylat (ca. 70 %, Emulan^{®} TO 4070, BASF AG, Ludwigshafen, DE); I: Polyoxyethylensorbitanmonolaurat | | | | | | |

**Tabelle 2**

| Schaum Nr. | Aufnahmegeschwindigkeit¹⁾ [s] | Freie Saugleistung [g/100 cm²] | MVTR [g/m²*24 h] |
|---|---|---|---|
| V1 | > 60 ²⁾ | 33,6 | 1493 |
| V2 | > 60 ³⁾ | 26,7 | n.b. |
| V3 | > 60 ⁴⁾ | 31,1 | n.b. |
| V10 | > 20⁴⁾ | n.b. | n.b. |

| | | | |
|---|---|---|---|
| ¹⁾ Zeit bis zum vollständigen Eindringen von einem Milliliter der Prüflösung A, hergestellt wie in DIN EN 13726-1, Teil 3.2, beschrieben; Prüfung der zum Papier gerichteten Seite; ²⁾ Messung unmittelbar; ³⁾ Messung nach 4 d Lagerung; ⁴⁾ Messung nach 1 d Lagerung | | | |

### Beispiele S1-S5: Herstellung von Schäumen aus der Polyurethan-Dispersion 1 und Imprauil^{®} DLU

Wie in Tabelle 3 angegeben, wurde die Polyurethan-Dispersion 1, hergestellt nach Beispiel 1, bzw. Impranil^{®} DLU mit verschiedenen (Schaum-) Additiven vermischt und unter Verwendung eines handelsüblichen Handrührgerätes (Rührer aus gebogenem Draht) auf 0,5 Liter Schaumvolumen aufgeschlagen. Danach wurden die Schäume mittels eines Filmziehgerätes (Rakel) mit Spalthöhe 6 mm auf nicht-haftendes Papier aufgezogen und unter den angegebenen Bedingungen getrocknet.

Es wurden durchweg reinweiße Schäume mit guten mechanischen Eigenschaften und einer feinen Porenstruktur erhalten. Wie der Tabelle 4 zu entnehmen ist, konnte durch Verwendung der speziellen (Schaum-) Additive die Aufnahmegeschwindigkeit von physiologischer Salzlösung erheblich verbessert werden (alle < 1 s). Alle Schäume zeigen darüber hinaus eine gute freie Saugleistung sowie hohe Wasserdampfdurchlässigkeit.

**Tabelle 3**

| | | (Schaum-) Additive | | | | | | |
|---|---|---|---|---|---|---|---|---|
| Schaum Nr. | Polyurethan-Dispersion 1 | Typ¹⁾ | Menge [g] | Typ¹⁾ | Menge [g] | Typ¹⁾ | Menge [g] | Aushärtung |
| S1 | 117,5 | C | 0,5 | J | 6,1 | - - - | - - - | 60 min 60 °C, |
| S2 | 127,1 | C | 0,5 | J | 6,1 | - - - | - - - | 10 min 120 °C |
| S3 | 120,0 | C | 0,90 | J | 6,1 | K | 0,27 | 10 min 120 °C |
| S4 | 120,0 | C | 0,23 | J | 3,1 | K | 0,14 | |
| | | | | | | | | |

| | | (Schaum-) Additive | | | | | | |
|---|---|---|---|---|---|---|---|---|
| Schaum Nr. | Impranil^{®} DLU | Typ³⁾ | Menge [g] | Typ³⁾ | Menge [g] | Typ³⁾ | Menge [g] | Aushärtung |
| S5 | 120,0 | C | 0,23 | J | 3,1 | K | 0,14 | 10 min 120 °C |

| | | | | | | | | |
|---|---|---|---|---|---|---|---|---|
| ¹⁾ C: Sulfobernsteinsäurebis(2-ethylhexylester), Na-Salz; J: Alkylpolyglycosid auf Basis von Dodecylalkohol (ca. 52 %, Simulsol^{®} SL 26, Seppic GmbH, Köln, DE); K: Natriumstearat | | | | | | | | |

**Tabelle 4**

| Schaum Nr. | Aufnahmegeschwindigkeit¹⁾ [s] | Freie Saugleistung [g/100 cm²] | MVTR [g/m^{2*}24h] |
|---|---|---|---|
| S1 | 1²⁾ | 32,1 | 1900 |
| | 1³⁾ | | |
| S2 | 1³⁾ | 32,0 | n.b. |
| S3 | 1 | 31,3 | n.b. |
| S4 | 1 | 42,0 | 3900 |
| S5 | 1³⁾ | 43,4 | n.b. |

| | | | |
|---|---|---|---|
| 1) Zeit bis zum vollständigen Eindringen von einem Milliliter der Prüflösung A, hergestellt wie in DIN EN 13726-1, Teil 3.2, beschrieben; Prüfung der zum Papier gerichteten Seite; ²⁾ Messung unmittelbar; ³⁾ Messung nach 1 d Lagerung | | | |

### Beispiel S6: Herstellung, hydrophiler Polyurethan-Schäume mit geringen Mengen Ammoniumstearat

120 g der nach Beispiel 1 hergestellten Polyurethan-Dispersion 1 wurden mit 1,47 g Plantacarec® 1200 UP*) (mittels Zitronensäure zuvor auf pH 7 eingestellt) und 0,24 g Stokal® STA vermischt. Unter Verwendung eins Handrührgerätes wurde die Mischung dann in 20 Minuten auf ein Schaumvolumen von 500 ml aufgeschäumt. Danach wurde der Schaum mittels eines Rakels (Spalthöhe: 6 mm) auf nicht-haftendes Papier appliziert und getrocknet (20 Min. bei 120°C).
*⁾ Alkylpolyglycosid auf Basis von C12- bis C16-Alkoholen, ca. 50 Gew.-% in Wasser, Cognis GmbH & Co. KG, Düsseldorf, DE

Es wurde ein reinweißer, feinzelliger, hydrophiler Schaum erhalten (Absorption von 1 ml der Prüflösung A in weniger als 3 Sekunden).

## Patentansprüche

1. Verwendung von Alkylpolyglycosiden als Stabilisatoren für Polyurethanschäume.

2. Verwendung gemäß Anspruch 1, **dadurch gekennzeichnet, dass** neben der Stabilisierung auch eine Hydrophilierung der Schäume erreicht wird.

3. Verwendung gemäß Anspruch 1 oder 2, **dadurch gekennzeichnet, dass** es sich bei den Polyurethanschäumen um solche handelt, wie sie durch physikalische Trocknung aus wässrigen Polyurethandispersionen erhalten werden.

4. Verwendung gemäß einem der Ansprüche 1 bis 3, **dadurch gekennzeichnet, dass** als Alkylpolyglycoside solche der Formel (I) eingesetzt werden:

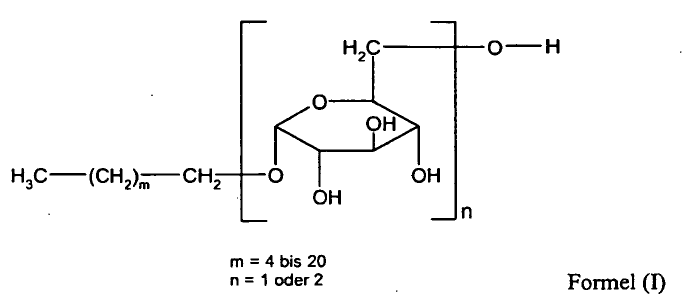

5. Zusammensetzungen enthaltend wässrige, anionisch hydrophilierte Polyurethan-Dispersionen (I) und Schaumadditive (II), wobei diese Schaumadditive (II) wenigstens ein Alkylpolyglycosid umfassen.

6. Zusammensetzungen gemäß Anspruch 5, **dadurch gekennzeichnet, dass** die darin enthaltenen wässrigen, anionisch hydrophilierten Polyurethan-Dispersionen (I) erhältlich sind, indem
A) isocyanatfunktionelle Prepolymere mindestens aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und/oder gegebenenfalls nichtionischen Hydrophilierungsmitteln,
hergestellt werden,
B) deren freie NCO-Gruppen dann ganz oder teilweise
B1) gegebenenfalls mit aminofunktionellen Verbindungen mit Molekulargewichten von 32 bis 400 g/mol und/oder
B2) isocyanatreaktiven, bevorzugt aminofunktionellen, anionischen oder potentiell anionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

7. Zusammensetzungen gemäß Anspruch 5 oder 6, **dadurch gekennzeichnet, dass** bei der Herstellung der wässrigen, anionisch hydrophilierten Polyurethan-Dispersionen (I) in A1) 1,6-Hexamethylendiisocyanat, Isophorondiisocyanat, die isomeren Bis-(4,4'-isocyanatocyclohexyl)methane, sowie deren Mischungen eingesetzt werden und in A2) eine Mischung aus Polycarbonatpolyolen und Polytetramethylenglykolpolyolen eingesetzt wird, wobei der Anteil der Summe der Polycarbonat- und Polytetramethylenglykolpolyetherpolyole an der Komponente A2) mindestens 70 Gew.-% beträgt.

8. Zusammensetzungen gemäß einem der Ansprüche 5 bis 7, **dadurch gekennzeichnet, dass** als Alkylpolyglycoside solche der Formel (I) eingesetzt werden:

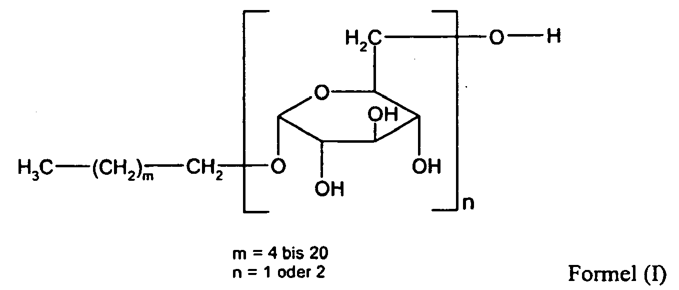

9. Zusammensetzungen gemäß einem der Ansprüche 5 bis 8, **dadurch gekennzeichnet, dass** neben den Alkylpolyglycosiden auch Ester der Sulfobernsteinsäure und/oder Alkali- oder Erdalkalimetallalkanoate.

10. Verfahren zur Herstellung von Polyurethan-Schäumen, bei dem eine Zusammensetzung gemäß einem der Ansprüche 5 bis 9 aufgeschäumt und getrocknet wird.

11. Polyurethanschäume erhältlich nach einem Verfahren gemäß Anspruch 10.

12. Verwendung der Polyurethanschäume gemäß Anspruch 11 als Wundauflage.

## Claims

1. Use of alkylpolyglycosides as stabilizers for polyurethane foams.

2. Use according to Claim 1, **characterized in that** the foams are hydrophilicized as well as stabilized.

3. Use according to Claim 1 or 2, **characterized in that** the polyurethane foams are obtained from aqueous polyurethane dispersions by physical drying.

4. Use according to any one of the Claims 1 to 3, **characterized in that** alkylpolyglycosides of formula (I) 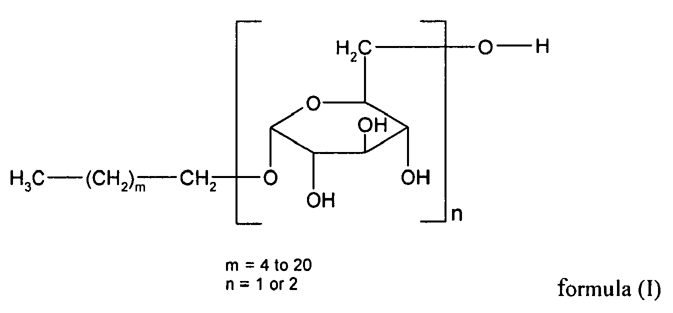 are used.

5. Compositions containing aqueous, anionically hydrophilicized polyurethane dispersions (I) and foam additives (II), these foam additives (II) comprising at least an alkylpolyglycoside.

6. Compositions according to Claim 5, **characterized in that** the aqueous, anionically hydrophilicized polyurethane dispersions (I) they contain are obtainable by
A) isocyanate-functional prepolymers being produced from
A1) organic polyisocyanates
A2) polymeric polyols having number-average molecular weights in the range from 400 to 8000 g/mol and OH functionalities in the range from 1.5 to 6 and
A3) optionally hydroxyl-functional compounds having molecular weights in the range from 62 to 399 g/mol and
A4) optionally isocyanate-reactive, anionic or potentially anionic and optionally nonionic hydrophilicizing agents
and
B) their free NCO groups then being wholly or partly reacted
B1) optionally with amino-functional compounds having molecular weights in the range from 32 to 400 g/mol and/or
B2) with isocyanate-reactive, preferably amino-functional, anionic or potentially anionic hydrophilicizing agents
by chain extension, and the prepolymers being dispersed in water before, during or after step B), any potentially ionic groups present being converted into the ionic form by partial or complete reaction with a neutralizing agent.

7. Compositions according to Claim 5 or 6, **characterized in that** the aqueous, anionically hydrophilicized polyurethane dispersions (I) are produced using in A1) 1,6-hexamethylene diisocyanate, isophorone diisocyanate, the isomeric bis-(4,4'-isocyanatocyclohexyl)methanes and also mixtures thereof and in A2) a mixture of polycarbonate polyols and polytetramethylene glycol polyols, the proportion of component A2) which is contributed by the sum total of the polycarbonate and polytetramethylene glycol polyether polyols being at least 70% by weight.

8. Compositions according to any one of the Claims 5 to 7, **characterized in that** alkylpolyglycosides of formula (I) 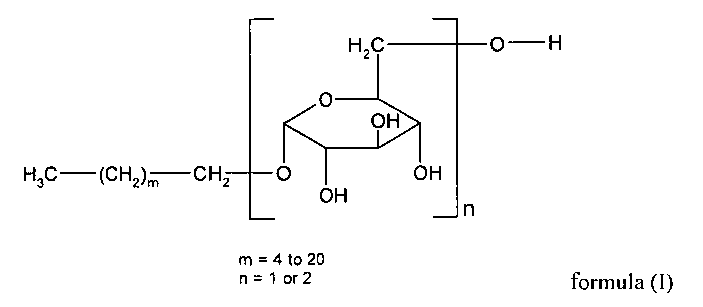 are used.

9. Compositions according to any one of the Claims 5 to 8, **characterized in that** esters of sulphosuccinic acid and/or alkali metal or alkaline earth metal alkanoates are used as well as alkylpolyglycosides.

10. Process for producing polyurethane foams, wherein a composition according to any one of the Claims 5 to 9 is frothed and dried.

11. Polyurethane foams obtainable by a process according to Claim 10.

12. Use of the polyurethane foams according to Claim 11 as a primary wound dressing.

## Revendications

1. Utilisation d'alkylpolyglycosides comme stabilisateurs pour des mousses de polyuréthane.

2. Utilisation selon la revendication 1, **caractérisée en ce qu'**outre la stabilisation, une hydrophilisation des mousses est également obtenue.

3. Utilisation selon la revendication 1 ou 2, **caractérisée en ce qu'**il s'agit, pour les mousses de polyuréthane, de mousses telles qu'elles sont obtenues par séchage physique à partir de dispersions aqueuses de polyuréthane.

4. Utilisation selon l'une quelconque des revendications 1 à 3, **caractérisée en ce qu'**on utilise comme alkylpolyglycosides ceux de formule (I) :

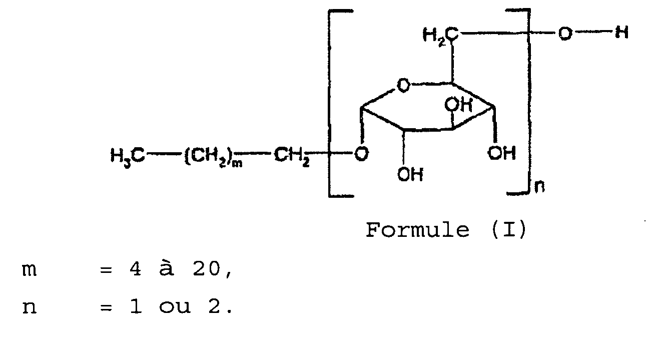

5. Compositions contenant des dispersions aqueuses de polyuréthane, hydrophilisées par voie anionique (I) et des additifs pour mousse (II), où ces additifs pour mousse (II) comprennent au moins un alkylpolyglycoside.

6. Compositions selon la revendication 5, **caractérisées en ce que** les dispersions aqueuses de polyuréthane, hydrophilisées par voie anionique (I) qui y sont contenues peuvent être obtenues **en ce que**
A) des prépolymères à fonctionnalité isocyanate sont préparés au moins à partir
A1) de polyisocyanates organiques
A2) de polyols polymères présentant des poids moléculaires numériques moyens de 400 à 8000 g/mole et des fonctionnalités OH de 1,5 à 6 et
A3) le cas échéant de composés à fonctionnalité hydroxy présentant des poids moléculaires de 62 à 399 g/mole et
A4) le cas échéant d'agents d'hydrophilisation réactifs avec isocyanate, anioniques ou potentiellement anioniques et/ou le cas échéant non ioniques,
B) dont les groupes NCO libres sont alors transformés totalement ou partiellement
B1) le cas échéant avec des composés à fonctionnalité amino présentant des poids moléculaires de 32 à 400 g/mole et/ou
B2) avec des agents d'hydrophilisation réactifs avec isocyanate, de préférence à fonctionnalité amino, anioniques ou potentiellement anioniques
avec allongement de chaîne et les prépolymères sont dispersés avant ou après l'étape B) dans l'eau, les groupes potentiellement ioniques le cas échéant contenus étant transformés en forme ionique par une transformation partielle ou totale avec un agent de neutralisation.

7. Compositions selon la revendication 5 ou 6, **caractérisées en ce qu'**on utilise, lors de la préparation des dispersions aqueuses de polyuréthane, hydrophilisées par voie anionique (I) dans A1) du 1,6-hexaméthylènediisocyanate, de l'isophoronediisocyanate, les bis-(4,4'-isocyanatocyclohexyl)méthanes isomères, ainsi que leurs mélanges et dans A2) un mélange de polycarbonate-polyols et de polytétraméthylèneglycolpolyols, où la proportion de la somme de polycarbonate-polyols et de polytétraméthylèneglycolpolyéther-polyols par rapport au composant A2) représente au moins 70% en poids.

8. Compositions selon l'une quelconque des revendications 5 à 7, **caractérisées en ce qu'**on utilise comme alkylpolyglycosides ceux de formule (I) :

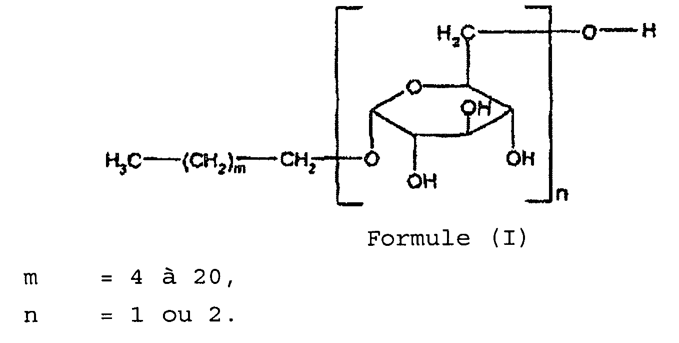

9. Compositions selon l'une quelconque des revendications 5 à 8, **caractérisées en ce qu'**outre les alkylpolyglycosides, on utilise également des esters de l'acide sulfosuccinique et/ou des alcanoates de métal alcalin ou alcalino-terreux.

10. Procédé pour la préparation de mousses de polyuréthane, dans lequel une composition selon l'une quelconque des revendications 5 à 9 est moussée et séchée.

11. Mousses de polyuréthane pouvant être obtenues selon un procédé selon la revendication 10.

12. Utilisation des mousses de polyuréthane selon la revendication 11 comme pansement.
